# EUROPEAN PATENT SPECIFICATION

(11) **EP 3 764 929 B1**
(45) Date of publication and mention of the grant of the patent: **18.10.2023**
(21) Application number: 19714951.1
(22) Date of filing: 18.03.2019
(51) Int. Cl.: A61B 17/24, A61M 31/00, A61M 25/00

(54) **SYSTEMS FOR TREATING THE NASAL CAVITY**
SYSTEME ZUR BEHANDLUNG DER NASENHÖHLE
SYSTÈMES POUR TRAITER LA CAVITÉ NASALE

(30) Priority: 16.03.2018 US 201862644137 P; 04.04.2018 US 201862652706 P
(43) Date of publication of application: 20.01.2021
(73) Proprietor: Arrinex, Inc., Menlo Park, CA 94025 (US)
(72) Inventor: FAHEY, Brian, Menlo Park, CA 94025 (US); FOX, William, Jason, San Mateo, CA 94402 (US); HESTER, Jerome, Redwood City, CA 94063 (US); SAADAT, Mojgan, Atherton, CA 94027 (US); SAADAT, Vahid, Atherton, CA 94027 (US)
(74) Representative: Röthinger, Rainer
(86) International application number: PCT/US2019/022802
(87) International publication number: WO 2019/178607

(56) References cited:
- US-A1- 2012 053 404
- US-B1- 6 398 758

## Description

### FIELD

The present technology is related to systems, devices, and methods for applying treatments within a nasal cavity. More specifically, the disclosed technology relates to providing adequate pain relief to allow interventions related to the nasal cavity (e.g., cryo-ablation, turbinate reduction, thermal ablation, mechanical reconstruction, thermal treatments, and tissue modifying procedure manipulates tissue) to be performed with minimal or no discomfort to patients. The technology is beneficial in allowing for nasal procedures to be performed more safely and with less morbidity, and as such allows for improvements in medical management of nasal and sinus conditions.

### BACKGROUND

Many nasal cavity related medical conditions (e.g., ailments including rhinitis and sinusitis and structural abnormalities such as a deviated septum) may be treated using instruments that are inserted into a nasal cavity. However, the nasal cavity is a highly-innervated region of tissue and as such is particularly sensitive to noxious stimuli. Patient discomfort is typically a consequence of instruments making even relatively mild contact with a wall of the nasal cavity. During interactions between instruments and the nasal or sinus cavities (e.g., during the application of a treatment or procedure to a tissue), discomfort may be significant. As such, it is a standard of care and general practice to apply an anesthetic agent or an analgesic to the nasal cavity prior to instrument insertion. During some procedures, it may be necessary to utilize a number of anesthetics and/or sedatives to achieve a more substantial degree of pain control.

However, there are several shortcomings and challenges associated with achieving pain control within the nasal cavity, particularly with applying anesthesia to the nasal cavity. For example, the nasal cavity is a relatively narrow passageway with limited space to maneuver the instruments. The surfaces of the nasal cavity are also highly-irregular in shape as there are numerous nooks and ridges that are created by the protrusions of turbinate bones and other surfaces in the nasal cavity. The positions of the sensory afferents in the nasal cavity are broadly distributed and may exist across a range of locations, not all of which may be easily accessible from a single conventional approach. These factors make uniform and complete application of anesthetic agents challenging.

There are a number of additional challenges associated with applying anesthetic agents to the nasal cavity. One such challenge is the tendency for a portion of the anesthetic substance to be exposed to the throat, which is a common occurrence associated with the use of liquids and sprays. For example, in some instances, when anesthetics reach the throat, a patient may experience a diminished throat sensation that may result in difficulty breathing and associated patient distress. However, with many anesthetic substances and methods/devices for application, it remains difficult to obtain thorough tissue coverage and thus achieve the desired broad pain-control effect without excess anesthetic reaching the throat.

Additional challenges relate to the business aspects of performing a procedure, particularly for in-office procedures. Within many areas of medicine there is a consistent pressure to reduce costs. Extraneous costs related to wasted anesthetic substances, overly expensive anesthetic delivery mechanisms, or increased time spent by clinicians to perform a procedure are generally viewed as undesirable. To achieve widespread adoption and to impact the most patients, solutions for adequate patient anesthesia should be cost-effective to implement and deliver.

Another challenge is the lack of simple yet effective ways to test and evaluate the thoroughness of anesthesia that is induced in the nasal cavity. Given a lack of options, modern medical practice may not involve testing for anesthetic depth or pain control. Any adjustments to anesthesia are often reactive (e.g., patient complaints or other indicators of pain prompt a practitioner to pause, withdraw instruments, and apply more of an anesthetic agent), which is a process that is considered burdensome from both time and cost perspectives and that results in unnecessary patient discomfort. Improved techniques would allow for proactive testing of anesthesia effectiveness and for any adjustments to be made prior to initiating procedures, allowing for improved patient comfort and reduced costs via a more efficient workflow.

Accordingly, there is a need to provide improved anesthetic delivery to the nasal cavity. Additionally, there is a need to allow for improved testing of the depth or effectiveness of anesthesia applied within the nasal cavity.

An apparatus and method for selectively delivering a medicament to a target location of the wall of a body cavity, blood vessel, or the like, as disclosed in document US 6,398,758 B1, comprises a compressed hollow cylinder of permeable, expandable foam, attached to the distal end of a delivery device such as a catheter or catheter guidewire. Using the delivery device, the compressed cylinder is advanced to a target location within the body, and the compressed foam cylinder is allowed to expand so as to contact the walls of the body cavity, blood vessel, or the like, while allowing bodily fluids to freely flow through the central lumen of the cylinder. The foam cylinder is provided with means for perfusing it with a medicament so that the medicament is placed in contact with the walls of the patient's anatomy, and is also provided with means for preventing the medicament from entering the patient's bloodstream.

### BRIEF SUMMARY

The invention is defined in claim 1. Further embodiments are defined in the dependent claims. No surgical methods form part of the invention. In an example, an apparatus for delivering an anesthetic agent to a target tissue in a nasal cavity of a patient is described. The apparatus includes an elongated shaft with a proximal end and a distal end. The apparatus also includes an absorbent plug coupled to the distal end of the elongated shaft, wherein the absorbent plug is configured to occupy a first volume in a compressed state and a second volume in an uncompressed state, wherein the second volume is greater than the first volume, and wherein the absorbent plug is configured to store an anesthetic agent and deliver the anesthetic agent to the target tissue in the nasal cavity by contacting the target tissue in the uncompressed state. The apparatus also includes a sheath at least partially covering the absorbent plug, wherein the sheath retains the absorbent plug in the compressed state when positioned at least partially over the absorbent plug, and wherein removal of the sheath allows for the absorbent plug to expand to the uncompressed state. A hand piece is coupled to the proximal end of the elongated shaft, wherein the hand piece is configured to facilitate gripping and manipulating the apparatus, and wherein the hand piece and the elongated shaft are configured to be decoupled from the absorbent plug after removal of the sheath.

The features, functions, and advantages that have been discussed can be achieved independently in various examples or may be combined in yet other examples further details of which can be seen with reference to the following description and drawings.

### BRIEF DESCRIPTION OF THE DRAWINGS

The novel features believed characteristic of the illustrative examples are set forth in the appended claims. The illustrative examples, however, as well as a preferred mode of use, further objectives and descriptions thereof, will best be understood by reference to the following detailed description of an illustrative example of the present disclosure when read in conjunction with the accompanying drawings, wherein:
Fig. 1 shows an anatomy of nerves and blood vessels in a nasal cavity, according to an example.
Fig. 2A shows an apparatus including an absorbent plug for applying an anesthetic agent, according to an example.
Fig. 2B shows the apparatus of Fig. 2A positioned in a nasal canal of a patient, according to an example.
Fig. 2C shows the apparatus of Fig. 2A where the elongated shaft is detachable from the absorbent plug structure via a removal mechanism, according to an example.
Fig. 3 shows another apparatus including an absorbent plug, according to an example.
Fig. 4 shows another apparatus including an anesthetic delivery lumen, according to an example.
Fig. 5 shows another apparatus including multiple lumens, according to an example.
Fig. 6 shows a method for achieving pain control in the nasal cavity, according to an example.
Fig. 7 shows a method for achieving pain control in the nasal cavity that can be used with the method shown in Fig. 6, according to an example.
Fig. 8 shows a method for achieving pain control in the nasal cavity that can be used with the method shown in Fig. 6, according to an example.
Fig. 9 shows a method for achieving pain control in the nasal cavity that can be used with the method shown in Fig. 6, according to an example.
Fig. 10 shows a method for achieving pain control in the nasal cavity that can be used with the method shown in Fig. 6, according to an example.

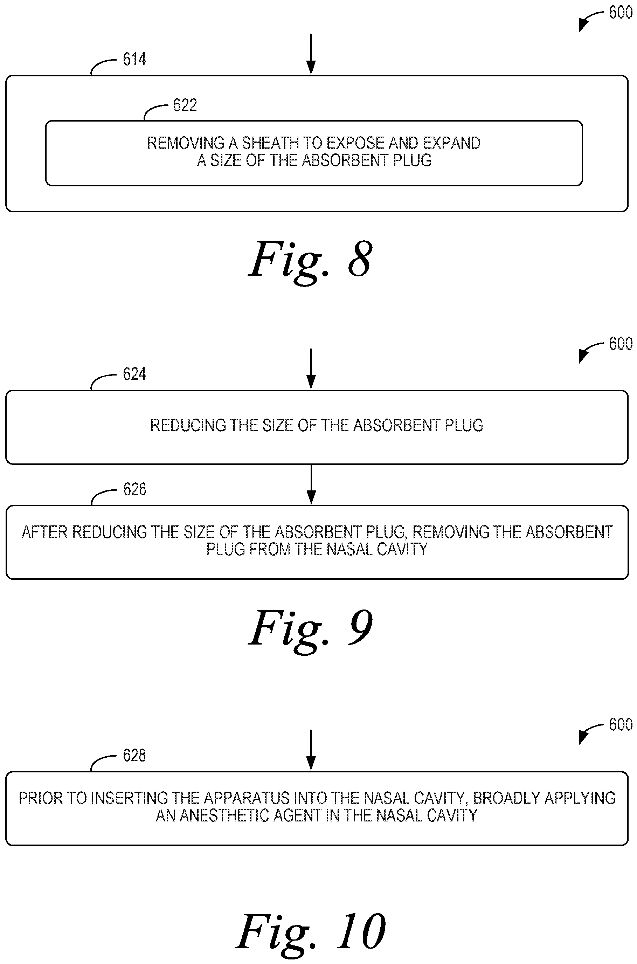

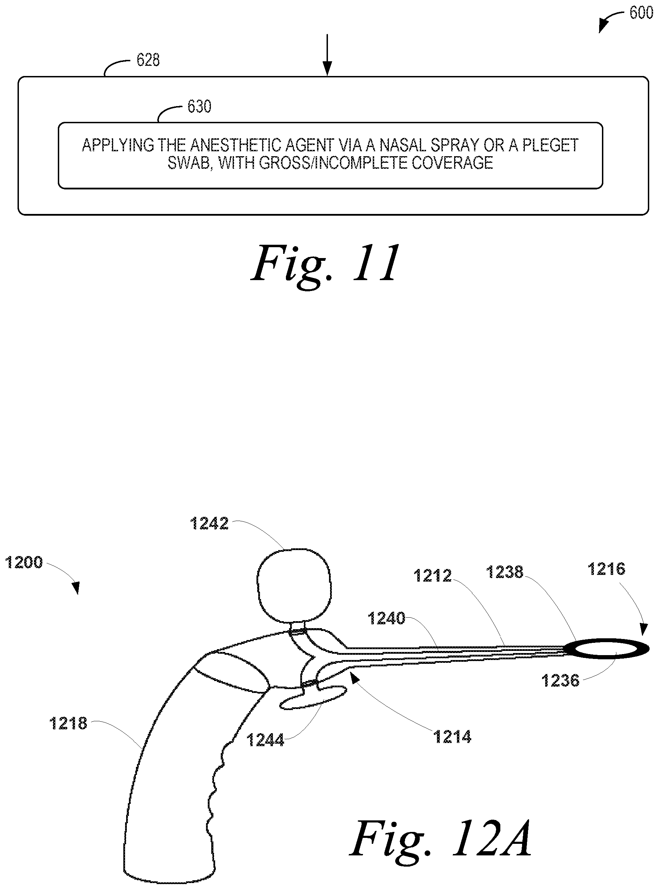

Fig. 10 shows a method for achieving pain control in the nasal cavity that can be used with the method shown in Fig. 11, according to an example.
Fig. 12A shows an apparatus for deploying an anesthetic agent via an expanding balloon, according to an example.

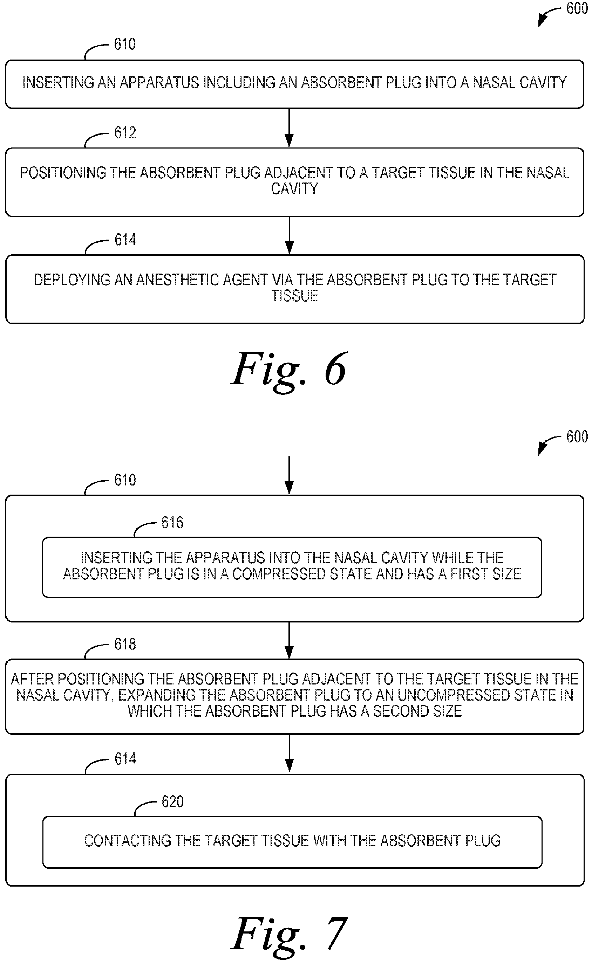

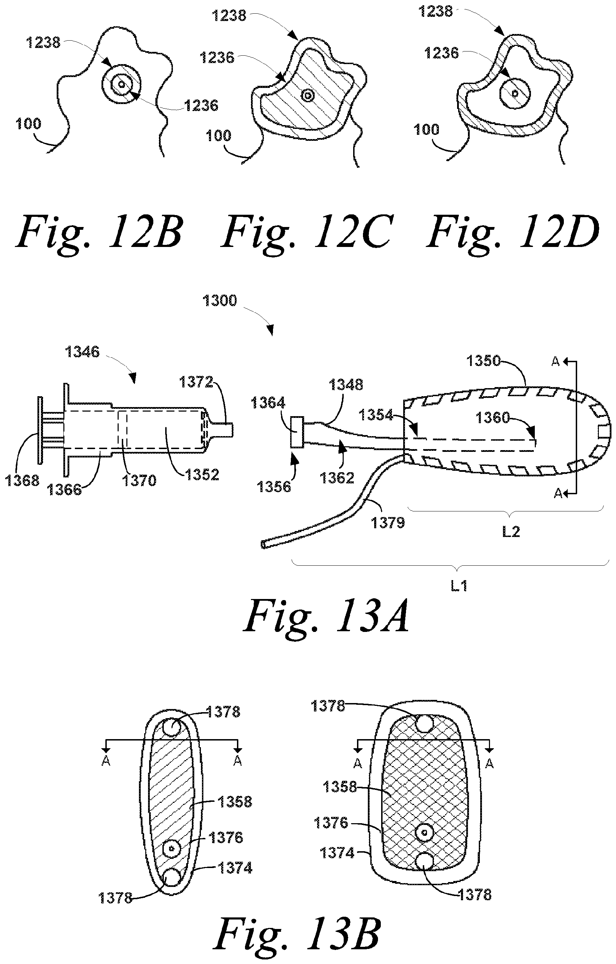

Fig. 12B shows a transverse view of the apparatus of Fig. 6A inserted into a nasal cavity with the expanding balloon in a deflated configuration, according to an example.
Fig. 12C shows a transverse view of the apparatus of Fig. 6A inserted into the nasal cavity with the expanding balloon in an inflated configuration, according to an example.
Fig. 12D shows a transverse view of the apparatus of Fig. 6A inserted into the nasal cavity with the expanding balloon in a deflated configuration but with the absorbent material remaining in contact with tissue of the nasal cavity, according to an example.
Fig. 13A shows an apparatus for deploying an anesthetic agent via an injectable expanding agent, according to an example.
Fig. 13B shows a side cross-sectional view of the apparatus of Fig. 7A taken along line A-A, according to an example.

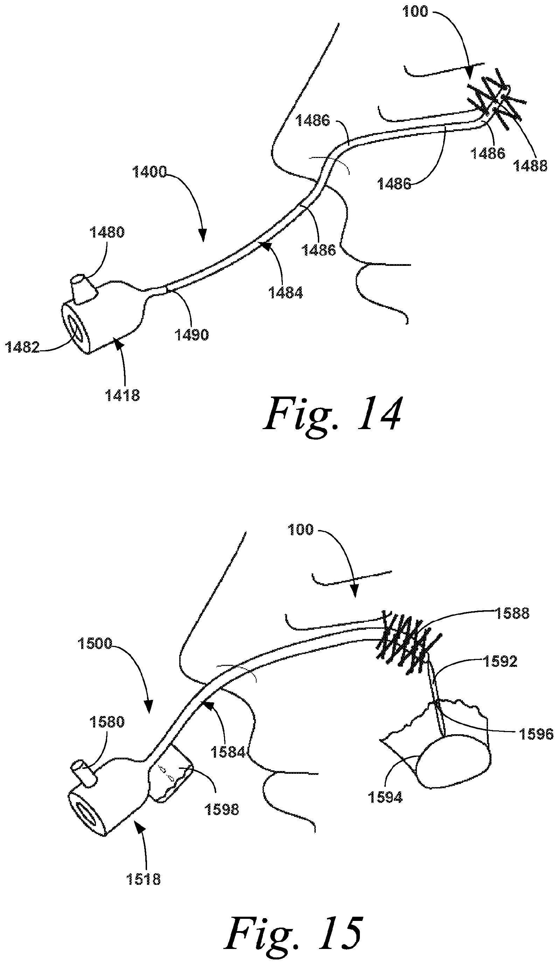

Fig. 14 shows an apparatus for deploying a substance to tissue using a catheter configured to spray a substance outward from a catheter body, according to an example.
Fig. 15 shows an apparatus for deploying a substance to tissue using a catheter configured to trap and remove excess substance, according to an example.

### DETAILED DESCRIPTION

The present technology is related to systems, devices, and methods for creating an anesthetic effect in a tissue. More specifically, the present technology relates to delivering anesthesia and creating an anesthetic effect in a tissue region including or proximal to a nasal cavity and/or a sinus cavity. This technology can be particularly useful when performing interventional procedures that would otherwise provide relatively moderate or severe patient discomfort, where a lack of adequate anesthesia would lead to patient morbidity, excess costs, and be discouraging to both practitioners and patients alike in terms of willingness to participate in the procedure in the future. Additionally, the technology of the present disclosure can enable time-efficient and cost-efficient implementation, and improve patient care from both clinician and patient perspectives.

In the following description, various examples of the present technology will be described. For purposes of explanation, specific configurations and details are set forth in order to provide a thorough understanding of the examples. However, it will also be apparent to one skilled in the art that the present technology can be practiced without the specific details. Furthermore, well-known features can be omitted or simplified in order not to obscure the example being described.

Various aspects of the technology described herein can be applied to any of the particular applications set forth below or for any other types of pain control systems or methods. The technology can be applied as a standalone system or method, or as part of an integrated medical treatment system.

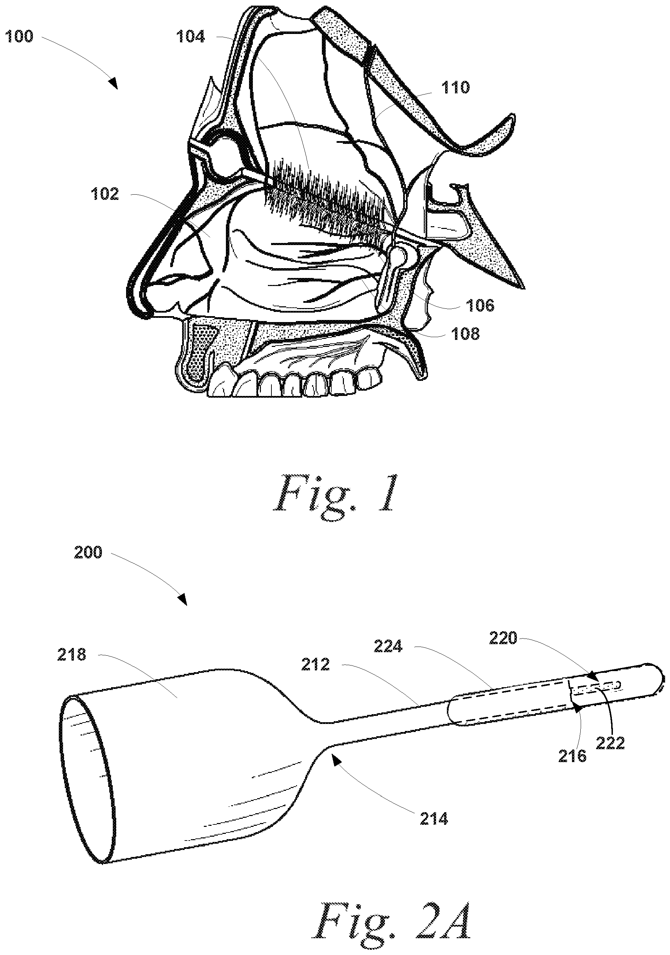

Fig. 1 illustrates an anatomy of a nasal cavity 100 and specifically indicates a location of a plurality of nerves of interest, according to an example. For example, Fig. 1 illustrates a lateral wall 102, one or more olfactory nerves 104, a lateral posterior superior nasal nerve 106, a lateral posterior inferior nasal nerve 108, and a sphenopalatine nerve 110. As shown in Fig. 1, the location of the nerves 104, 106, 108, 110 vary considerably in the different regions of the nasal cavity 100. As the systems and methods of the present disclosure are suitable to delivery to one or more of the nerves 104, 106, 108, 110, the systems and methods of the present disclosure can be configured to access and achieve pain control in one or more different regions of the nasal cavity 100.

Within examples, apparatuses for delivery of an anesthetic agent to the nasal cavity are described herein. One example apparatus includes an absorbent plug (also referred to herein as a tamponade), which can be inserted via the nostril and positioned within the nasal cavity (e.g., using a pleget or probe having an elongated shaft). The elongated shaft can be malleable and/or flexible to allow for navigation and placement within the tortuous anatomy of the nasal cavity. The absorbent plug can include a soft and absorbent material (such as, e.g., cotton or gauze), which allows for the absorbent plug to temporarily store and transport the anesthetic agent to an anatomical region for application (e.g., a region in the nasal cavity 100 shown in Fig. 1). When placed in contact with a target tissue, the absorbent plug can deliver the anesthetic agent to the target tissue in the nasal cavity and, thus, one or more target nerves within the target tissue. Within examples, longer contact times between the absorbent plug and the target tissue can lead to increased anesthetic application (i.e., relatively greater delivery of the anesthetic agent to the target tissue and target nerve(s) than when the absorbent plug is in contact with the target tissue for a relatively shorter amount of time).

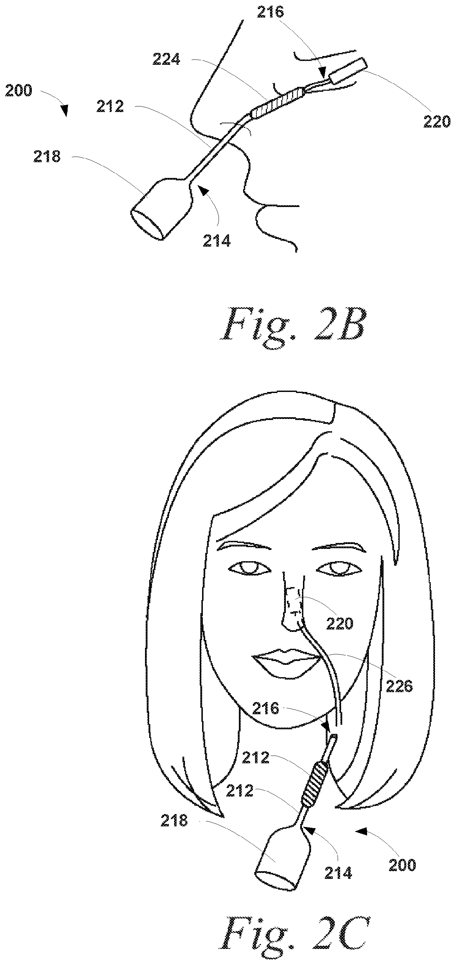

Figs. 2A-2B show an apparatus 200 for applying an anesthetic agent, according to an example. In particular, Fig. 2A shows the apparatus 200 in a first state that can facilitate inserting the apparatus 200 in a nasal cavity 100, and Fig. 2B shows the apparatus 200 in a second state that can facilitate delivering the anesthetic agent to one or more nerves (e.g., the nerves 104, 106, 108, 110) in the nasal cavity 100.

As shown in Figs. 2A-2B, the apparatus 200 includes an elongated shaft 212 having a proximal end 214 and a distal end 216. The elongated shaft 212 can include a malleable and/or flexible material. This can assist in inserting the apparatus 200 through a relatively narrow and irregularly shaped passageway of the nasal cavity 100 to position the distal end 216 at a target tissue in the nasal cavity 100. As noted above, the target tissue can include one or more target nerves (e.g., the nerves 104, 106, 108, 110) to which anesthesia is to be applied.

The proximal end 214 of the elongated shaft 212 is coupled to a hand piece 218. Within examples, the hand piece 218 is configured to facilitate gripping and manipulating the apparatus 200. For instance, in a dimension transverse to a longitudinal axis of the apparatus 200, the hand piece 218 can have a size that is relatively larger than a size of the elongated shaft 212 to provide a relatively larger gripping surface, which an operator can use to maneuver the apparatus 200 during insertion of the apparatus 200 in the nasal cavity 100.

As shown in Figs. 2A-2B, the apparatus 200 also includes an absorbent plug 220 at the distal end 216 of the elongated shaft 212. The absorbent plug 220 is configured to store an anesthetic agent 222 and deliver the anesthetic agent 222 to the target tissue in the nasal cavity 100 by contacting the target tissue. In an example, the absorbent plug 220 can include a soft and absorbent material capable of retaining the anesthetic agent 222 prior to contact with the target tissue. For instance, the anesthetic agent 222 can be in a liquid form and the absorbent plug 220 can include a plurality of fibers that can retain the anesthetic agent 222 via absorption prior to contact with the target tissue. Responsive to contact between the absorbent plug 220 and the target tissue, the fibers of the absorbent plug 220 can release the anesthetic agent 222 to deliver the anesthetic agent 222 to the target tissue. Also, within examples, the absorbent plug 220 can be pre-infused with the anesthetic agent 222 prior to the absorbent plug 220 being inserted into the nasal cavity 100 to deliver the anesthetic agent 222 to the target tissue, as described below.

Within examples, the apparatus 200 can also include a sheath 224 disposed on and translatable relative to the elongated shaft 212 between a first position shown in Fig. 2A and a second position shown in Fig. 2B. In general, the first position of the sheath 224 is distal of the second position of the sheath 224. As shown in Fig. 2A, when the sheath 224 is in the first position, the sheath 224 at least partially (or entirely) covers the absorbent plug 220. As such, when the sheath 224 is in the first position, the absorbent plug 220 is in a compressed state due to the sheath 224 applying a compressive force to the absorbent plug 220 (i.e., the sheath 224 covers at least a portion of the absorbent plug 220 so that the absorbent plug 220 is in the compressed state). In the compressed state, the absorbent plug 220 occupies a first volume (i.e., the absorbent plug 220 can have a first size). Thus, the sheath 224 can be configured to initially cover and compress the absorbent plug 220 in the first position, creating a slim profile (e.g., a cross-sectional diameter less than approximately 1 millimeter (mm)) that allows for improved navigation of the distal end 216 of the elongated shaft 212 in the nasal cavity 100.

To transition the apparatus 200 from the first state shown in Fig. 2A to the second state shown in Fig. 2B, the sheath 224 can be proximally translated from the first position to the second position. As shown in Fig. 2B, when the sheath 224 is in the second position, the sheath 224 is retracted proximally to expose at least a portion (or all) of the absorbent plug 220 such that the compressive force applied by the sheath 224 to the absorbent plug 220 is reduced or eliminated. As a result, when the sheath 224 is in the second position, the absorbent plug 220 is in an uncompressed state in which the absorbent plug 220 occupies a second volume, which is greater than the first volume (i.e., the absorbent plug 220 can have a second size, which is greater than the first size). Accordingly, in the uncompressed state, the absorbent plug 220 can expand to deliver the anesthetic agent 222 to a relatively larger surface area of the target tissue in the nasal cavity 100.

As described above, the absorbent plug 220 is configured to occupy the first volume in the compressed state and the second volume in the uncompressed state, where the second volume is greater than the first volume. In one example, the absorbent plug 220 can have a cross-sectional diameter that is less than approximately 1 mm in the compressed state and a cross-sectional diameter that is greater than approximately 1 mm in the uncompressed state. In other example, the absorbent plug 220 can have a cross-sectional diameter that is less than or equal to a cross-sectional diameter of the elongated shaft 212 when the absorbent plug 220 is in the compressed state, and a cross-sectional diameter that is greater than the cross-sectional diameter of the elongated shaft 212 when the absorbent plug 220 is in the uncompressed state.

In operation, the apparatus 200 initially can be in the first state shown in Fig. 2A prior to insertion in the nasal cavity 100. As shown in Fig. 2A, the sheath 224 at least partially covers the absorbent plug 220 such that the absorbent plug 220 in the compressed state. While the sheath 224 is in the first position and the absorbent plug 220 is in the compressed state, the apparatus 200 can be inserted in the nasal cavity 100. For example, with or without endoscopic or fluoroscopic guidance, an operator can use the hand piece 218 to insert the distal end 216 of the elongated shaft 212 into the nasal cavity 100 until the distal end 216 of the elongated shaft 212 is proximate to a target tissue in a nasal cavity 100 of a patient. Because the sheath 224 at least partially covers and compresses the absorbent plug 220, inserting the distal end 216 with the sheath 224 in the first position can reduce (or minimize) the size of the absorbent plug 220 and thereby reduce (or minimize) contact between the apparatus 200 and the nasal cavity 100 during insertion to the target tissue. This can beneficially reduce (or minimize) patient discomfort and improve (or maximize) operational efficiencies.

Additionally, with the sheath 224 in the first position and the sheath 224 at least partially covering the absorbent plug 220 so that the absorbent plug 220, the sheath 224 can help to reduce (or minimize) contact between the absorbent plug 220 and the anesthetic agent 222 and the nasal cavity 100 during insertion to the target tissue. This can beneficially help to reduce (or minimize) delivering the anesthetic agent 222 to portions of the nasal cavity 100 that are outside of the target tissue.

After the apparatus 200 is inserted in the nasal cavity 100 and positioned at the target tissue, the sheath 224 can be translated relative to the elongated shaft 212 and the absorbent plug 220 in a direction from the distal end 216 of the elongated shaft 212 toward the proximal end 214 of the elongated shaft 212 (i.e., from the first position to the second position). Fig. 2B shows the apparatus 200 after the apparatus 200 is inserted in the nasal cavity 100 and the sheath 224 is translated from the first position to the second position. As shown in Fig. 2B, the sheath 224 in the second position does not cover the absorbent plug 220 and the absorbent plug 220 is in the uncompressed state. That is, by translating the sheath 224 from the first position to the second position, the sheath 224 can be retracted to uncover and deploy the absorbent plug 220. In turn, this allows the absorbent plug 220 to expand in the region of interest and contact the target tissue. This contact will deliver an anesthetic agent 222 to the target tissue.

After translating the sheath 224 from the first position to the second position, the apparatus 200 can be maintained in position for a prescribed period of time to allow for the anesthetic agent 222 to release from the absorbent plug 220 and be delivered to the target tissue (e.g., approximately 5 minutes to approximately 15 minutes). In some implementations, it may be desirable to maintain the absorbent plug 220 in contact with the target tissue for a relatively long period of time. In one example, the apparatus 200 can be configured to enhance patient comfort during the time for delivering the anesthetic agent 222 to the target tissue.

For instance, as shown in Fig. 2C, the apparatus 200 is configured such that, after translating the sheath 224 to the second position, the hand piece 218 and the elongated shaft 212 are decoupled from the absorbent plug 220 and removed from the nasal cavity 100. This can beneficially allow the absorbent plug 220 to remain at the target tissue in the nasal cavity 100 while reducing (or minimizing) a weight and/or a size of a remainder of the apparatus 200 in the nasal cavity 100.

Additionally, for example, decoupling the elongated shaft 212 and the hand piece 218 can facilitate removing the sheath 224. For instance, as shown in Fig. 2C, when the sheath 224 is in the second position, the sheath 224 is located on the elongated shaft 212. As such, when the elongated shaft 212 is decoupled from the absorbent plug 220, the sheath 224 can be removed with elongated shaft 212.

As illustrated in Fig. 2C, in one example, decoupling the elongated shaft 212 from the absorbent plug 220 can expose a removal member 226. In general, the removal member 226 extends from the absorbent plug 220 to a location external to the nasal cavity 100. In this way, the removal member 226 can provide a structure that can be used to remove the absorbent plug 220 from the nasal cavity 100 after the anesthetic agent 222 has been delivered to the target tissue. For instance, the removal member 226 can be a string that the operator can pull to remove the absorbent plug 220 from the nasal cavity 100.

Within examples, the removal member 226 can be a relatively soft material that has a relatively high tensile strength (e.g., a tensile strength that is sufficient to provide for pulling the absorbent plug 220 out of the nasal cavity 100). The relatively softness of the removal member 226 can improve comfort for the patient while the absorbent plug 220 is at the target tissue for the prescribed time for delivering the anesthetic agent 222 (e.g., several minutes). Additionally, the relatively high tensile strength of the removal member 226 can facilitate pulling on the removal member 226 in manner that can overcome a radial outward force of the absorbent plug 220 without detaching or breaking.

In one example, the removal member 226 can be housed in a lumen or a cavity within the elongated shaft 212 and/or the hand piece 218 when the apparatus 200 is in the first state and/or the second state (e.g., prior to decoupling the elongated shaft 212 from the absorbent plug 220).

In the example shown in Figs. 2A-2C, the sheath 224 is configured to translate in a proximal direction from the first position to the second position to expose the absorbent plug 220 and cause the absorbent plug 220 to expand. Additionally, in Figs. 2A 2C, the sheath 224 can be removed by decoupling the elongated shaft 212 from the absorbent plug 220. In another example, the sheath 224 can be additionally or alternatively removable from the elongated shaft 212 without decoupling the elongated shaft 212 from the absorbent plug 220.

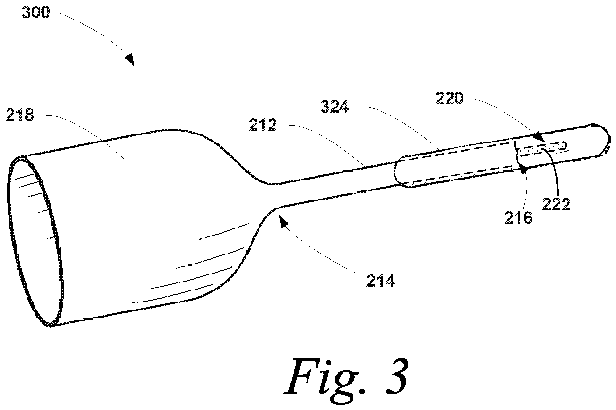

Fig. 3 depicts an apparatus 300 having a sheath 324 that can be moved without decoupling the elongated shaft 212 from the absorbent plug 220, according to an example. The apparatus 300 is substantially similar or identical to the apparatus 200 described above, except the sheath 324 is a removable barrier. The removable barrier of the sheath 324 can be a flexible sheet (e.g., a plastic wrapping) that can be removed after insertion of the absorbent plug 220 in the nasal cavity 100 to the target tissue.

In one implementation, the removable barrier of the sheath 324 can be a dissolvable coating covering the absorbent plug 220. In operation, the absorbent plug 220 at least partially covered by the removable barrier of the sheath 324 can be inserted in the nasal cavity 100 to the target tissue and then held in place at the target tissue until at least one (i) the dissolvable coating of the removable barrier dissolves due to moisture present within the nasal cavity 100, (ii) a liquid introduced by the operator dissolves the removable barrier, or (iii) the anesthetic agent 222 within the absorbent plug 220 dissolves the removable barrier.

In this way, the removable barrier of the sheath 324 can retain the absorbent plug 220 in the compressed state during insertion in the nasal cavity 100 and then, at the target tissue, be removed from the absorbent plug 220 (e.g., by dissolving or unwrapping) to allow for the absorbent plug 220 to expand to the uncompressed state.

In the examples described above, the apparatus 200, 300 includes the absorbent plug 220, which (i) retains the anesthetic agent 222 prior to insertion of the apparatus 200, 300 in the nasal cavity 100, and then (e.g., after translation and/or removal of the sheath 224, 324) (ii) is exposed to contact and deliver the anesthetic agent 222 to the target tissue in the nasal cavity 100. In other examples, the apparatus 200, 300 can retain and/or deliver the anesthetic agent 222 in additional or alternative manners.

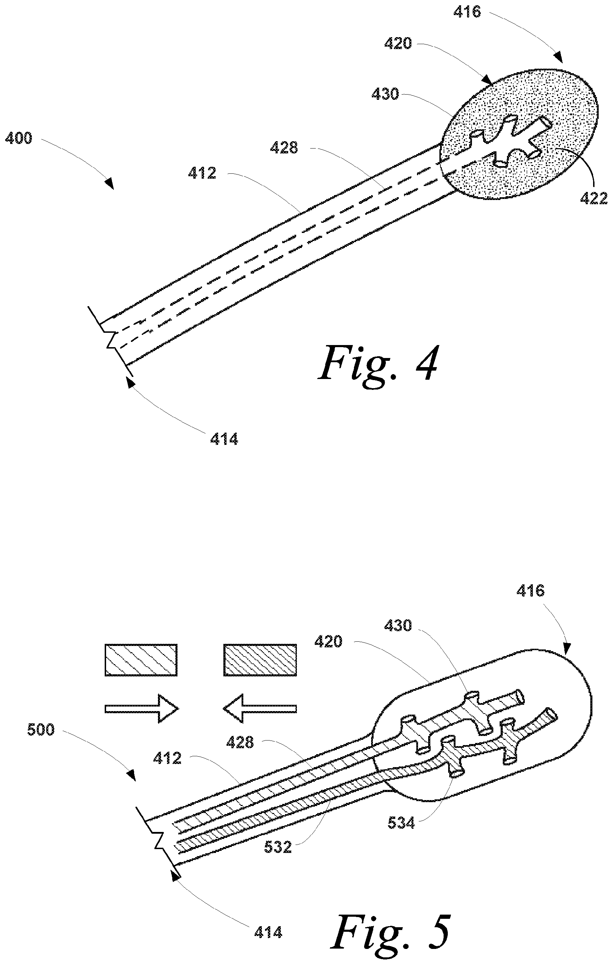

Fig. 4 illustrates an apparatus 400 that can deliver an anesthetic agent 422, according to another example. As shown in Fig. 4, the apparatus 400 includes an elongated shaft 412 having a proximal end 414 and a distal end 416. The apparatus 400 can optionally include a hand piece (e.g., the hand piece 218) coupled to the proximal end 414 of the elongated shaft 412, as described above. Additionally, the elongated shaft 412 can be made from a flexible and/or malleable material, as described above.

The apparatus 400 also includes an absorbent plug 420 coupled to the distal end 416 of the elongated shaft 412. As described above, the absorbent plug 420 is configured to store the anesthetic agent 222 and deliver the anesthetic agent 222 to the target tissue in the nasal cavity 100 by contacting the target tissue.

Additionally, as shown in Fig. 4, the apparatus 400 can include a lumen 428 extending through the elongated shaft 412 and into the absorbent plug 420. The lumen 428 can include one or more ports 430 within the absorbent plug 420 and configured to deliver the anesthetic agent 422 into the absorbent plug 420 so that the anesthetic agent 422 be absorbed by the absorbent plug 420.

As shown in Fig. 4, the one or more ports 430 can open in a region of the absorbent plug 420. The absorbent plug 420 can be comprised of a porous material (e.g., an open-cell foam, a closed-cell foam, and/or a sponge-like material) that can allow the anesthetic agent 222 to flow from the one or more ports 430 at an internal portion of the absorbent plug 420 to an external surface of the absorbent plug 420, which is configured to contact the target tissue. In one example, the absorbent plug 420 has a density that allows for the absorbent plug 420 to be soft and conformable (which may help to reduce or minimize a risk of tissue injury) while also ensuring that the absorbent plug 420 has a structural integrity to remain firmly in place even as a fluid, gel, foam, or other substance is delivered to the absorbent plug 420.

In use, the elongated shaft 412 of the apparatus 400 can be maneuvered by an operator such that the distal end 416, including the absorbent plug 420, is proximate to the target tissue to which it is desired to apply the anesthetic agent 422. The operator can then deploy the anesthetic agent 422, for example, by injecting the anesthetic agent 422 using a syringe (not shown) into the proximal end 414 of the elongated shaft 412 via the lumen 428. The anesthetic agent 422 flows through the lumen 428 from the proximal end 414 to the distal end 416 and out of the one or more ports 430 into the absorbent plug 420. The anesthetic agent 422 then flows through the absorbent plug 420 from the one or more ports 430 to the external surface of the absorbent plug 420, which contacts the target tissue in the nasal cavity 100. As a result of this contact, the absorbent plug 420 delivers the anesthetic agent to the target tissue in the nasal cavity 100.

In one example, the absorbent plug 420 is adapted for insertion in the nasal cavity 100 in a stable, reduced-size configuration. In such an example, the absorbent plug 420 can be composed of a dry and compressed sponge-like material which allows for a relatively small diameter (e.g., a cross-sectional diameter less than approximately 1 mm) to facilitate navigation within the nasal cavity 100 and positioning of the absorbent plug 420 in a desired region at the target tissue. Once positioned at the target tissue, the absorbent plug 420 can begin to expand as the absorbent plug 420 absorbs moisture (e.g., moisture from mucosal tissues of the nasal cavity 100). Responsive to the anesthetic agent 422 injected in the lumen 428 contacting the absorbent plug 420, the absorbent plug 420 can further expand. After the absorbent plug 420 expands, the absorbent plug 420 can establish a contact pressure with the target tissue in the nasal cavity 100 and thereby deliver the anesthetic agent 422 to the target tissue.

In additional or alternative examples, the apparatus 200, 300, 400 can include one or more removal mechanisms to assist with removing the absorbent plug 220, 420 after the anesthetic agent 222, 422 is applied. For instance, after the absorbent plug 420 has expanded to a full size (e.g., responsive to proximally translating the sheath 224 from the first position to the second position, removing the sheath 324, and/or injecting the anesthetic agent 422 via the lumen 428), the absorbent plug 220, 420 can apply a radial outward force to adjacent tissues in the nasal cavity 100. While this force can assist with effective delivery of the anesthetic agent 222, 422, the radial outward force can make the absorbent plug 220, 420 difficult and/or uncomfortable to remove from the nasal cavity 100.

Fig. 5 depicts an apparatus 500 that includes an additional removal mechanism to assist with removing an absorbent plug from the nasal cavity 100, according to an example. In Fig. 5, the apparatus 500 is substantially similar to the apparatus 400 shown in Fig. 4. For example, the apparatus 500 includes the elongated shaft 412 having the proximal end 414 and the distal end 416. Additionally, the apparatus 500 includes the lumen 428 extending through the elongated shaft 412 and into the absorbent plug 420. Further, the lumen 428 includes the one or more ports 430 in the absorbent plug 420.

As shown in Fig. 5, the apparatus 500 further includes a second lumen 532 extending through the elongated shaft 412 and into the absorbent plug 420. As described above, the lumen 428 is configured to deliver the anesthetic agent 422 into the absorbent plug 420, and the absorbent plug 420 then delivers the anesthetic agent 422 to the target tissue.

Additionally, as shown in Fig. 5, the apparatus 500 includes a second lumen 532 extending through the elongated shaft 412 and into the absorbent plug 420. The second lumen 532 is configured to couple to a suction source (e.g., a vacuum source and/or a negative pressure source) at the proximal end 414 and/or proximal to a hand piece coupled to the proximal end 414 (e.g., the hand piece 218). The second lumen 532 also includes one or more second ports 534 within the absorbent plug 420. In this arrangement, the suction source can apply, via the second lumen 532 and the one or more second ports 534, suction to the absorbent plug 420 to remove the anesthetic agent 422 from the absorbent plug 420 and actuate the absorbent plug 420 from an uncompressed state to a compressed state (i.e., to reduce a size of the absorbent plug 420).

More particularly, to remove the absorbent plug 420 from the nasal cavity 100, residual fluid in the absorbent plug 420 can be extracted via the one or more second ports 534 and withdrawn through the second lumen 532 into a fluid reservoir (not shown), which is fluidly coupled with the second lumen 532 at the proximal end 414 and/or proximal of the hand piece. As noted above, the residual fluid in the absorbent plug 420 can be removed from the absorbent plug 420 by, for example, applying a negative pressure (e.g., a vacuum and/or a suction force) to the second lumen 532. As the residual fluid is extracted from the absorbent plug 420, the negative pressure can reduce the size of the absorbent plug 420, which can allow for easier withdrawal of the absorbent plug 420 from the nasal cavity 100 compared to withdrawing a fully expanded absorbent plug 420 from the nasal cavity 100.

In some examples, the anesthetic agent 222, 422 can be a fluid-based anesthetic agent. However, in other examples, the anesthetic agent 222, 422 can include one or more foams, gases, mists, and/or gels. Within examples, the lumen 428 and/or the second lumen 532 can be configured to supply and/or withdraw, respectively, the anesthetic agent 222, 422 and/or residual substances in the absorbent plug 220, 520 including one or more fluids, foams, gases, mists, and/or gels.

Additionally, although Fig. 5 depicts the second lumen 532 in the apparatus 500 that is similar to the apparatus 400 shown in Fig. 4 (e.g., including the lumen 428), the second lumen 532 can additionally or alternatively be incorporated into the apparatus 200 shown in Fig. 2 and/or the apparatus 300 shown in Fig. 3 to facilitate removal of the absorbent plug 220 from the nasal cavity 100.

Referring now to Fig. 6, a flowchart for a process 600 for controlling pain in a nasal cavity is illustrated, according to example. As shown in Fig. 6, the process 600 can include inserting an apparatus including an absorbent plug into a nasal cavity at block 610. At block 612, the process 600 can include positioning the absorbent plug adjacent to a target tissue in the nasal cavity. At block 614, the process 600 can include deploying an anesthetic agent via the absorbent plug to the target tissue.

Figs. 7-8 depict additional aspects of the process 600 according to further examples. As shown in Fig. 7, inserting the apparatus into the nasal cavity at block 610 can include inserting the apparatus into the nasal cavity while the absorbent plug is in a compressed state and has a first size at block 616. Additionally, as shown in Fig. 7, the process 600 can include, after positioning the absorbent plug adjacent to the target tissue in the nasal cavity, expanding the absorbent plug to an uncompressed state in which the absorbent plug has a second size at block 618. The second size of the absorbent plug in the uncompressed state is greater than the first size of the absorbent plug in the compressed state. Also, as shown in Fig. 7, deploying the anesthetic agent at block 614 can include contacting the target tissue with the absorbent plug at block 620.

As shown in Fig. 8, positioning the absorbent plug adjacent to the target tissue at block 614 can include removing a sheath to expose and expand a size of the absorbent plug at block 622. As shown in Fig. 9, the process 600 can also include reducing the size of the absorbent plug at block 624 and, after reducing the size of the absorbent plug at block 624, removing the absorbent plug from the nasal cavity at block 626.

As shown in Fig. 10, prior to inserting the apparatus into the nasal cavity at block 610, the process 600 can include broadly applying an anesthetic agent in the nasal cavity at block 628. As shown in Fig. 11, broadly applying the anesthetic agent in the nasal cavity at block 628 can include applying the anesthetic agent via a nasal spray or a pleget swab, with gross/incomplete coverage at block 630. In some instances, performing block 630 may not provide adequate pain control for a given type of procedure by itself, but may improve patient comfort during navigation of the apparatus, an endoscope, and/or another instrument in the nasal cavity.

In the examples described above, the absorbent plug 220, 420 provides an applicator that can expand to conform to an irregular shape of the nasal cavity 100 and deliver the anesthetic agent 222, 422 to the target tissue. In some implementations, the absorbent plug 220, 420 can expand passively. In other implementations, an apparatus can include an actively expandable applicator that can be inserted in the nasal cavity 100 with a relatively small size and then actively expanded to conform to the irregular shape of the nasal cavity 100 and deliver the anesthetic agent. For instance, within examples, an outward force from an expanding agent can allow for improved conformability to irregularly shaped surfaces and improved contact with mucosal tissues.

Referring now to Fig. 12A, an apparatus 1200 for delivering an anesthetic agent (e.g., the anesthetic agent 222, 422) to a target tissue in the nasal cavity 100 of a patient is shown. The apparatus 1200 includes an elongated shaft 1212 with a proximal end 1214 and a distal end 1216. The elongated shaft 1212 can include a flexible and/or malleable material, as described above.

The apparatus 1200 also includes a balloon 1236 coupled to the distal end 1216 of the elongated shaft 1212. The balloon 1236 is configured to be inflated such that the balloon 1236 expands outward from the elongated shaft 1212. As such, the balloon 1236 can have (i) a deflated state in which the balloon 1236 has a first size and (ii) an inflated state in which the balloon 1236 has a second size, which is greater than the first size. As one example, the balloon 1236 can be a compliant latex balloon.

Additionally, the apparatus 1200 includes an absorbent sheath 1238 covering the balloon 1236. The absorbent sheath 1238 is configured to store an anesthetic agent and deliver the anesthetic agent to a target tissue in the nasal cavity 100 by contacting the target tissue. For instance, the absorbent sheath 1238 can retain and release the anesthetic agent in manner similar to the absorbent plug 220 described above.

To inflate and deflate the balloon 1236, the apparatus 1200 includes an air delivery lumen 1240, an air pump 1242, and a release valve 1244. In Fig. 12A, the air pump 1242 and the release valve 1244 are coupled to a hand piece 1218. The air delivery lumen 1240 has a first end coupled to the balloon 1236 and a second end coupled to the air pump 1242 and the release valve 1244.

In this arrangement, the absorbent sheath 1238 and the balloon 1236 can be inserted into the nasal cavity 100 and positioned adjacent to the target tissue with the balloon 1236 in the deflated state. After the absorbent sheath 1238 and the balloon 1236 are positioned adjacent to the target tissue in the nasal cavity 100, the air pump 1242 can be activated to supply air through the air delivery lumen 1240 in the elongated shaft 1212. Within examples, the air pump 1242 can be manually operated and/or electrically operated.

Responsive to the air pump 1242 supplying the air to the balloon 1236, the balloon 1236 expands from the deflated state to the inflated state and, thus, causes the absorbent sheath 1238 to expand and contact the target tissue in the nasal cavity 100 so as to deliver the anesthetic agent to the contacted target tissue. Until the release valve 1244 is actuated, the release valve 1244 inhibits (or prevents) the air egressing from the balloon 1236 (i.e., the release valve 1244 ensures that air flow is unidirectional toward to the distal end 1216 of the elongated shaft 1212).

After the absorbent sheath 1238 has contacted the target tissue for sufficient to time to deliver the anesthetic agent to the target tissue, the release valve 1244 can be actuated to return the balloon from the inflated state to the deflated state. Responsive to the balloon 1236 reducing in size (e.g., in the deflated state), the balloon 1236 and the absorbent sheath 1238 can be withdrawn from the nasal cavity 100. Thus, by inserting and withdrawing the balloon 1236 and the absorbent sheath 1238 while the balloon is in the deflated state, patient discomfort can be reduced (or minimized).

Figs. 12B-12D illustrate transverse views of the balloon 1236 and absorbent sheath 1238 in various states within an irregular-shaped region of the nasal cavity 100, according to an example. In use, an anesthetic agent can be applied to the absorbent sheath 1238, for example, by soaking the absorbent sheath 1238 in a fluid, applying a gel to the absorbent sheath 1238, and/or applying a foam to the absorbent sheath 1238.

As described above, the balloon 1236 and the absorbent sheath 1238 can be inserted into the nasal cavity 100 with the balloon 608 in the deflated state such that the balloon 1236 and the absorbent sheath 1238 have a relatively slim profile, as shown in Fig. 12B. With the distal end 1216 of the elongated shaft 1212 positioned such that the balloon 1236 is at the target tissue, the operator can operate the air pump 1242 (e.g., by manually squeezing the air pump 1242) to cause the balloon 1236 to inflate. In one example, the operator can operate the air pump 1242 to inflate the balloon 1236 to the inflated state by manually squeezing the air pump 1242. In other examples, the balloon 1236 can be inflated by additional or alternative mechanisms such as, for instance, via a syringe of saline, or any other mechanism known in the art for inflating a balloon.

As shown in Fig. 12C, when the balloon 1236 is in the inflated state, the compliant nature of the balloon 1236 can allow the balloon 1236 to conform to an irregularly-shaped surface in the nasal cavity 100 and, thereby, force the absorbent sheath 1238 into contact with the target tissue in the nasal cavity. That is, the balloon 1236 can help to force the absorbent sheath 1238 into relatively continuous contact with the target tissue over most or an entirety of the irregularly-shaped surface.

The absorbent sheath 1238 can be held in contact with target tissue via the balloon 1236 in the inflated state for a predetermined period of time to achieve an anesthetic effect, as shown in Fig. 12C. Following this period of time, the release valve 1244 can be activated by the operator to deflate the balloon 1236 and return the distal end 1216 of the elongated shaft 1212 to the slim profile to facilitate removal of the apparatus 1200 from the nasal cavity 100. Other methods of deflating the balloon 1236 prior to removal are possible as well.

In one example, the absorbent sheath 1238 can be mildly adhesive or otherwise have a means to stay in contact with the target tissue even after the balloon 1236 is deflated, as shown in Fig. 12D. For example, the absorbent sheath 1238 containing the anesthetic agent can be detachable from the balloon 1236. After detaching the absorbent sheath 1238 from the balloon 1236, the elongated shaft 1212 of the apparatus 1200 can be removed from the nasal cavity 100 while the absorbent sheath 1238 (which includes the anesthetic agent) remains in the nasal cavity 100. This approach can, for instance, improve patient comfort.

In one implementation, the balloon 1236 can decouple from the absorbent sheath 1238 responsive to deflation of the balloon 1236. Additionally, within examples, the apparatus 1200 can include a removal member (e.g., the removal member 226) that can facilitate removing the absorbent sheath 1238 from the nasal cavity 100 following application of the anesthetic agent. In one example, the removal member can include a string and/or braided wire coupled to the absorbent sheath 1238, and the removable member can be exposed responsive to the balloon 1236 decoupling from absorbent sheath 1238.

In the example shown in Figs. 12A-12B, the apparatus 1200 includes a single balloon 1236. However, in other examples, the apparatus 1200 can include a plurality of balloons 1236 to expand an anesthetic carrying layer (e.g., the absorbent sheath 1238) and contact the target tissue. For example, in one implementation, the apparatus 1200 can include 2-5 relatively small balloons 1236 to increase conformability to irregularly-shaped regions of the nasal cavity 100. In some implementations, the balloons 1236 can inflate together as a group or, in other implementations, each balloon 1236 can be inflated independently of the other balloons 1236 (e.g., by adjusting various one or more valves and/or knobs on the hand piece 1218 that control one or more regions of the apparatus 1200 receive air inflow from the air pump 1242).

Fig. 13A illustrates an example for systems, devices, and methods to apply anesthesia using an expanding agent. In Fig. 13A, a system 1300 for delivering an anesthetic agent can include dispensing tool 1346, a delivery cannula 1348, expandable member 1350, and an expanding agent 1352.

The delivery cannula 1348 and the expandable member 1350 can have a combined length, L1, (e.g., along a longitudinal axis) between approximately 100 mm and approximately 200 mm. The delivery cannula 1348 is coupled to the expandable member 1350 at an interface 1354. In an example, the delivery cannula 1348 can be constructed of a semi-rigid plastic or metal material, and the delivery cannula 1348 can have an outer surface that is rigid between a proximal end 1356 and the interface 1354 with the expandable member 1350.

At the interface 1354, the delivery cannula 1348 is coupled to the expandable member 1350 superficially and inserts into an internal layer 1358 (Layer 3) of the expandable member 1350, as shown in Fig. 13B. Within examples, the delivery cannula 1348 inserts approximately 5 mm to approximately 100 mm into the expandable member 1350. If the delivery cannula 1348 is inserted more than approximately 20 mm into the expandable member 1350, a distal end 1360 of the delivery cannula 1348 can become flexible. The delivery cannula 1348 can contain a central lumen 1362 that extends along an entire length of the delivery cannula from the proximal end 1356 to the distal end 1360. The proximal end 1356 can include a mating feature 1364 (e.g., a luer) that is configured to couple with the dispensing tool 1346.

Within examples, the dispensing tool 1346 can have a design similar to a syringe. For example, the dispensing tool 1346 can include a container body 1366 that defines an internal chamber in which the expanding agent 1352 is contained. Additionally, for example, the dispensing tool 1346 can include a plunger 1368 having a stopper 1370 that is axially movable in the internal chamber to dispense the expanding agent 1352 from a dispense end 1372 of the dispensing tool 1346. The dispense end 1372 is configured to couple with the central lumen 1362 at the mating feature 1364. As examples, the expanding agent 1352 can include saline, a gel, and/or an activating fluid that mixes with a mating fluid and thereby causes the internal layer 1358 to expand.

Within examples, the expandable member 1350 is actuatable between a collapsed state and an expanded state. In one implementation, when the expandable member 1350 is in the collapsed state, the expandable member 1350 can be approximately 30 mm to approximately 100 mm in length (L2) along the longitudinal axis, less than approximately 2 mm in thickness, and approximately 5 mm to approximately 20 mm in height. Also in this implementation, when the expandable member 1350 is in the expanded state, the thickness of the expandable member 1350 can be greater than approximately 3 mm, the height can be at least 20% greater than the height of the expandable member 1350 in the collapsed state, and the length can remain approximately the same as in the collapsed state (i.e., the expandable member 1350 can have a negligible change in the length (L2)).

Within examples, the expandable member 1350 can be constructed of an absorbent outer layer 1374 (Layer 1) that is configured to absorb a therapeutic agent or mixture of agents. As examples, the absorbent outer layer 1374 can include one or more of a cotton, a terry cloth, and/or a sponge-like material. Also, within examples, the absorbent outer layer 1374 can have a thickness of approximately 0.25 mm to approximately 0.5 mm.

The absorbent outer layer 1374 can surround the expandable member 1350 on at least three sides. An intermediate layer 1376 (Layer 2) of the expandable member 1350 can be constructed of a thin film that inhibits (or prevents) fluid from passing through the intermediate layer 1376 to more internal layers (e.g., the internal layer 1358) for a period of time (e.g., several minutes).

Within examples, the intermediate layer 1376 can have a thickness that is less than approximately 0.005 mm and be constructed from a compliant polymer (such as, e.g., latex/urethane) or from a dissolvable substance (such as, e.g., sugar or Kollidon). As shown in Fig. 13B, the intermediate layer 1376 encapsulates the internal layer 1358, which is an expanding layer. Within examples, the internal layer 1358 can include a highly conforming expanding material (such as, e.g., a foam, an open-cell sponge, and/or a gel). In some examples, the internal layer 1358 can also be empty and serve as a fillable reservoir for fluid. Also, in some examples, the internal layer 1358 in the collapsed state is less than approximately 1 mm thick and, in some examples, the internal layer 1358 can be less than approximately 0.5 mm thick.

As shown in Fig. 13B, the internal layer 1358 can include a malleable spine 1378 that provides the expandable member 1350 with push-ability and structure. The malleable spine 1378 can be comprised of a material (such as, e.g., copper, bronze, and/or annealed stainless steel) that can be formed into a desired shape by the user, for example a shape matching the anatomy of a region of the nasal cavity 100. Within examples, the malleable spine 1378 can extend along an entire length of the expandable member 1350, or the malleable spine 1378 can extend along an entire length of a perimeter of the internal layer 1358 only.

As shown in Fig. 13B, the system 1300 can include a tether 1379 that is coupled to a proximal end of the malleable spine 1378 and configured to assist in removing the expandable member 1350 after the expandable member 1350 is deployed in the nasal cavity 100. The tether 1379 can be composed of a suture-like material that can withstand tension (e.g., a material such as Kevlar and/or other suturing materials). When the expandable member 1350 is expanded via the introduction of the expanding agent 1352, the internal layer 1358 can swell stretching the absorbent outer layer 1374 until the expandable member 1350 meets a rigid surface, at which time the internal layer 1358 presses and conforms to a surface such that the absorbent outer layer 1374 (which contains a therapeutic agent) is contacts the target tissue in the nasal cavity 100. Within examples, a radial outward force applied by the expandable member 1350 on target tissue is tailored to conform to the target tissue without causing meaningful tissue displacement.

In examples of a method for use of a system, for example as illustrated in Figures 13A-13B, can include the following steps: (1) the expandable member 1350 is coated or soaked in a therapeutic agent such that it can hold the agent and deliver it to a target tissue; (2) the expanding agent 1352 is loaded into the dispensing tool 1346, which interfaces with the delivery cannula 1348; (3) the expandable member 1350 is configured into a desired shape by adjusting the malleable spine 1378 and advanced into the nasal cavity 100; (4) while the expandable member 1350 is at the target tissue, the expanding agent 1352 is dispensed into the internal layer 1358 of the expandable member 1350, which expands and conforms to the target tissue in the nasal cavity; (5) the operator removes the delivery cannula 1348 from the nasal cavity 100, leaving the expandable member 1350 behind in the nasal cavity 100; (6) after the patient is sufficiently anesthetized, the operator pulls on tether 1379 until the expandable member 1350 exits the nasal cavity 100.

In additional or alternative examples, a catheter can be used that can spray an anesthetic agent directly into one or more regions of interest. Within examples, the catheter can eject the anesthetic agent with a force to project the anesthetic agent against tissue walls in the nasal cavity 100. Also, within examples, the catheter can reduce (or minimize) an amount of the anesthetic agent that reaches a patient's throat. Accomplishing either of these stated objectives represents a marked improvement over devices and techniques in the prior art, which tend to be deployed by syringes or other low pressure mechanisms, leading to inaccurate placement, drippage, and the eventually migration of the anesthetic agent to the patient's throat.

Fig. 14 shows a catheter 1400 configured for use to achieve pain control in the nasal cavity 100, according to an example. The catheter 1400 includes a handle region 1418 that includes a loading port 1480 and a compressed air intake port 1482 configured to adapt to replaceable cartridges of compressed air (not shown). As shown in Fig. 14, the catheter 1400 can include a catheter shaft 1484, which can have a plurality of independently steerable sections 1486 that allow for a shape of the catheter shaft 1484 to be adjusted by the operator and allow for the catheter shaft 1484 to be positioned in various aspects of the nasal cavity. A distal end of the catheter 1400 has one or more output ports 1488 that allow for dispensing of an anesthetic agent.

Within examples, the catheter shaft 1484 can be disposable. Also, within examples, the catheter shaft 1484 can be coupled to the handle region 1418 by a threaded connection 1490 and/or using another connection means known to those skilled in the art.

In examples, the loading port 1480 is configured to receive a liquid anesthetic agent (e.g., via a luer fitting or another configuration known to those skilled in the art). The anesthetic agent can then flow from the loading port 1480 to the one or more output ports 1488 at the distal end of the catheter 1400 via an internal lumen (not shown). The one or more output ports 1488 (and/or one or more channels leading to the one or more output ports 1488) are shaped and configured to convert a liquid anesthetic agent into a fine mist as pressured air released into the air intake port 1482 drives the anesthetic agent out of the catheter 1400 towards the target tissue. This combination of outward spraying pressure and fine mist achieves multiple objectives: (a) allowing for application of the anesthetic agent even to regions in the nasal cavity 100 that the catheter 1400 cannot contact directly; (b) ensuring broad coverage due to the dispersive nature of the mist; and (c) due to the fine nature of the mist and the associated surface tension of the mist particles interacting with the target tissue. The drippage of the anesthetic agent into unwanted regions outside the target tissue (e.g., towards the patient's throat) can be avoided provided that a limited amount of the anesthetic agent is applied in each region.

In examples, the one or more output ports 1488 can be fixed to release the anesthetic agent in a stable set of directions relative to the catheter 1400. In examples, the catheter shaft 1484 and/or the one or more output ports 1488 can move either automatically or manually by the operator to adjust angles and/or directions at which the anesthetic agent is released from the one or more output ports 1488.

In examples, the air intake port 1482 can be configured to interface with a hand-pump or other source of compressed air. In examples, the air intake port 1482 can be replaced by a syringe/plunger mechanism or other suitable mechanism for producing the pressure gradient to drive an anesthetic through the catheter lumen and out of the one or more output ports 1488.

In examples, the catheter 1400 is configured to dispense the anesthetic agent including one or more of a gel, a foam, and/or other forms. In examples, the one or more output ports 1488 may not be configured to produce a mist, but can instead be configured to use the pressure generated by the catheter 1400 to deploy the anesthetic agent to various regions.

Referring now to Fig. 15, a catheter 1500 is shown according to another example. The catheter 1500 can be substantially similar to the catheter 1400 described with respect to Fig. 14. For example, the catheter 1400 can include a handle region 1518, a catheter shaft 1584, one or more output port 1586, an intake port 1582, and a loading port 1580 as described above.

Additionally, as shown in Fig. 15, the catheter 1500 includes a thin extension 1592 that can be deployed from the distal end of the catheter shaft 1584 using a dial (not shown) on the handle region 1518 or other adjustment mechanism. The thin extension 1592 includes an occlusion balloon 1594 at a distal end of thin extension 1592, and a suction port 1596 located between the occlusion balloon 1594 and the distal end of the catheter 1500. The suction port 1596 can assist in moving a substance through a lumen in the thin extension 1592 back into the catheter 1400 and ultimately into a waste reservoir 1598 located proximate to the handle region 1518. By using a hand-pump or operating a valve near the hand piece (not shown), the occlusion balloon 1594 can be inflated and deflated by the operator.

The catheter 1500 can be particularly useful when deploying anesthesia in certain patient anatomical regions. For example, as shown in Fig. 15, the distal end of the catheter 1500 includes the one or more output ports 1588 that can be positioned at a target tissue in the nasal cavity 100 where it is desired to apply anesthesia. After the one or more output ports 1588 are positioned at the target tissue, the thin extension 1592 can be deployed and the occlusion balloon 1594 can be inflated. The occlusion balloon 1594 then expands until the occlusion balloon 1594 contacts the target tissue. This contact between the occlusion balloon 1594 and the target tissue can act as a seal that effectively isolates a region of the catheter 1500 from tissue regions more distal, for example, the patient's throat.

The anesthetic agent (e.g., a mist or liquid spray) can be sprayed onto the target tissue via the one or more output ports 1588, and excess anesthetic agent can migrate downstream and be trapped by the occlusion balloon 1594. Once applying the anesthetic agent is complete, the suction port 1596 can be used to remove excess anesthetic agent from a region at the occlusion balloon 1594 and the excess anesthetic agent can be supplied through the catheter 1500 to the waste reservoir 1598. At the waste reservoir 1598, the excess anesthetic agent can be collected by the operator and discarded. The occlusion balloon 1594 can then be deflated, and the thin extension 1592 can then be retracted into the catheter 1400.

Examples can include various combinations of the approaches described above. As one illustrative example, a balloon or tamponade (e.g., an absorbent plug) approach can be first implemented to achieve bulk anesthetic effect. This can be followed by a mist or spray or gel/foam application to fine-tune the anesthetic effect and ensure coverage of particularly irregularly-shaped regions of tissue.

In examples, devices can include temperature-sensitive components that assist with deployment. In examples, a tamponade structure is condensed within a temperature-sensitive polymer casing. After placement in the nasal cavity in contact with the mucosal tissue, the polymer begins to warm as it absorbs heat from the body. Once it reaches a pre-determined temperature, the polymer begins to break-down, and the tamponade can move from its condensed size to a larger size that better conforms with the region of interest.

In examples, vacuum suction can be used. For example, a tamponade structure at the distal end of an insertion probe can include small openings in one or more locations, said openings being connected to an airflow lumen that traverses the length of the insertion probe and connects to a negative pressure source, for example a mild suction unit. In alternative examples, this lumen connects to a manual air pump and valve system that allows a user to create and maintain a negative pressure until a valve is adjusted to release this negative pressure. Negative pressure created at the openings in the tamponade structure will create a mild vacuum effect that draws the tamponade surface and the tissue surface closer to one another. This process can improve contact of the tamponade to the tissue surface, particularly in tissue regions that have irregular shape. The mild negative pressure/vacuum applied to soft tissues can stretch tissue membranes in such a way that it improves the penetration of anesthetics or other therapeutic agents. This latter feature can allow for shorter procedure time during the application of anesthesia.

Examples of systems and methods can include the application of cold air to the nasal cavity prior to and/or during procedures. Studies have demonstrated that some pain sensations driven by afferents in the nasal cavity, for example the sensation commonly referred to as the "ice cream headache," are inhibited in cold weather. During some procedures, for example cryoblation procedures that involve the application of cold temperatures to tissues, a possible side effect is patient discomfort similar to that of the ice cream headache. Applying cold temperature air to the nasal cavity can minimize or eliminate the potential manifestation of these types of discomfort. In examples, thin nasal cannula commonly used in clinical practice can be inserted into the nasal cavity to provide chilled low-flow oxygen for the patient to breath.

In examples, a therapeutic agent such as an anesthetic can be combined with a carrier molecule substance, for example a chemical compound similar to or with similar properties to dimethyl sulfoxide (DMSO). This combination allows for more rapid and/or more complete absorption of the agent by tissue, further increasing the utility of the technology described herein.

In examples, delivery of an anesthetic or other substance can be enhanced via energy delivery methods such as ultrasound, iontophoresis, or electrophoresis. These energy methods can use mild pressure waves or electrical currents to help widen tissue pores, increase local blood flow, allow for vasodilation, drive ions across membranes, or cause other effects that can aid in the more rapid or complete absorption of therapeutic agents into tissue.

In examples, a nasal cavity tool configured for the delivery of an anesthetic also includes an ultrasound transducer. Upon insertion, the ultrasound transducer is configured to transmit ultrasound energy into tissues targeted for treatment with an anesthetic. In examples, ultrasound frequencies of 5 - 15 MHz will be used in conjunction with local spatial peak, temporal average intensities of 0.1 - 100 W/cm². Ultrasound energy can be applied to tissue prior to, during, or following the application of a substance to tissue (or in any combination of these time periods) in order to enhance the delivery of the substance.

In examples, devices can be used to insulate the nasal cavity from noxious stimuli induced from contact with various tools. This can be accomplished with or without the use of anesthetic agents in combination. In examples, a small catheter contains a detachable and expandable foam tube at its distal end. This foam tube is initially held in a small diameter (1 - 5 mm) collapsed state by an introducer sheath. After insertion into the nasal cavity, the operator can remove the introducer sheath, allowing the foam tube to expand and make contact with nasal cavity walls. The insertion tool can then be removed to allow different tools to enter the nasal cavity. In examples, the foam tube has a thin wall of approximately 1 mm in diameter and a wider open central lumen of approximately 5 mm in diameter to allow the passage of tools through this lumen. In examples, the foam material has a consistency that is not irritating to the nasal cavity walls yet robust enough to insulate the walls from the sensation of a nasal tool or device making incidental contact with the foam. With the foam bumper in place, tools can be inserted into the nasal cavity with less patient discomfort as incidental contact with the sides of the nasal cavity will no longer produce an irritating sensation. Upon completion of a procedure, the foam bumper can be removed from the nasal cavity. In examples, the foam bumper is comprised of a bioabsorbable material that is naturally absorbed or otherwise flushed out by the body at a time following the procedure.

In examples, the foam bumper is manually expanded using for example a balloon catheter which can be inflated to expand the bumper and then removed from the nasal cavity once the bumper is in place. In examples, the bumper can be comprised of other soft, mechanically-insulating materials aside from foam - for example, a cotton or terrycloth material.

From the foregoing, it will be appreciated that specific examples of the technology have been described herein for purposes of illustration, but that various modifications can be made without deviating from the scope of the various examples of the technology. Further, while various advantages associated with certain examples of the technology have been described above in the context of those examples, other examples can also exhibit such advantages, and not all examples need necessarily exhibit such advantages to fall within the scope of the invention. Accordingly, the invention is not limited, except as by the appended claims.

The teachings of the technology provided herein can be applied to other systems, not necessarily the system described above. The elements and acts of the various examples described above can be combined to provide further implementations of the technology. Some alternative implementations of the technology can include not only additional elements to those implementations noted above, but also can include fewer elements. Further any specific numbers noted herein are only examples; alternative implementations can employ differing values or ranges, and can accommodate various increments and gradients of values within and at the boundaries of such ranges. Furthermore, the described features, advantages, and characteristics of the present technology can be combined in any suitable manner in one or more examples. One skilled in the relevant art will recognize that the present technology can be practiced without one or more of the specific features or advantages of a particular example. In other instances, additional features and advantages can be recognized in certain examples that cannot be present in all examples of the present technology.

The description of the different advantageous arrangements has been presented for purposes of illustration and description, and is not intended to be exhaustive or limited to the examples in the form disclosed. Many modifications and variations will be apparent to those of ordinary skill in the art. Further, different advantageous examples may describe different advantages as compared to other advantageous examples. The example or examples selected are chosen and described in order to explain the principles of the examples, the practical application, and to enable others of ordinary skill in the art to understand the disclosure for various examples with various modifications as are suited to the particular use contemplated.

## Claims

1. An apparatus (200, 400, 500) for delivering an anesthetic agent (222, 422) to a target tissue in a nasal cavity (100) of a patient, the apparatus (200, 400, 500) comprising:
an elongated shaft (212, 412) with a proximal end (214, 414) and a distal end (216, 416);
an absorbent plug (220, 420) coupled to the distal end (216, 416) of the elongated shaft (212, 412), wherein the absorbent plug (220, 420) is configured to occupy a first volume in a compressed state and a second volume in an uncompressed state, wherein the second volume is greater than the first volume, and wherein the absorbent plug (220, 420) is configured to store an anesthetic agent (222, 422) and deliver the anesthetic agent (222, 422) to the target tissue in the nasal cavity (100) by contacting the target tissue in the uncompressed state; and
a sheath (224) at least partially covering the absorbent plug (220, 420), wherein the sheath (224) retains the absorbent plug (220, 420) in the compressed state when positioned at least partially over the absorbent plug (220, 420), and wherein removal of the sheath (224) allows for the absorbent plug (220, 420) to expand to the uncompressed state;
**characterized in that** the apparatus (200, 400, 500) further comprises
a hand piece (218) coupled to the proximal end (214, 414) of the elongated shaft (212, 414), wherein the hand piece (218) is configured to facilitate gripping and manipulating the apparatus (200, 400, 500); and wherein the hand piece (218) and the elongated shaft (212, 412) are configured to be decoupled from the absorbent plug (220, 420) after removal of the sheath (224).

2. The apparatus (200) of claim 1, wherein the absorbent plug (220) is pre-infused with the anesthetic agent (222) prior to an insertion into the nasal cavity (100) to deliver the anesthetic agent (222).

3. The apparatus (200, 400, 500) of any one of claims 1 or 2, wherein the sheath (224) is translatable relative to the elongated shaft (212, 412) and the absorbent plug (220, 420), wherein the sheath (224) is translatable in a direction from the distal end (216, 416) toward the proximal end (214, 414) from a first position to a second position,
wherein, in the first position, the sheath (224) covers at least a portion of the absorbent plug (220, 420) so that the absorbent plug (220, 420) is in the compressed state, and
wherein, in the second position, the sheath (224) does not cover the absorbent plug (220, 420) and the absorbent plug (220, 420) is in the uncompressed state.

4. The apparatus (200, 400, 500) of any one of claims 1 to 3, the sheath (224) comprises a dissolvable coating at least partially covering the absorbent plug (220, 420) in the compressed state.

5. The apparatus (400, 500) of any one of claims 1 to 4, further comprising:
a lumen (428) extending through the elongated shaft (412) and into the absorbent plug (420), wherein the lumen (428) comprises a one or more ports (430) within the absorbent plug (420) configured to deliver the anesthetic agent (422) into the absorbent plug (420) to be absorbed by the absorbent plug (420).

6. The apparatus (400, 500) of claim 5, wherein the absorbent plug (420) is configured to expand responsive to the anesthetic agent (422) being absorbed by the absorbent plug (420).

7. The apparatus (500) of any one of claims 5 or 6, further comprising:
a second lumen (532) extending through the elongated shaft (412) and into the absorbent plug (420), wherein the second lumen (532) comprises one or more second ports (534) within the absorbent plug (420) configured to apply suction to remove the anesthetic agent (422) from the absorbent plug (420) and shrink the absorbent plug (420) from an uncompressed state to a compressed state.

8. The apparatus (200, 400, 500) of any one of claims 1 to 7, wherein the elongated shaft (212, 412) comprises a malleable and/or flexible material.

9. The apparatus (200, 400, 500) of any one of claims 1 to 8, wherein the absorbent plug (220, 420) comprises a soft and absorbent material capable of retaining the anesthetic agent (222, 422) prior to contacting the target tissue.

10. The apparatus (200, 400, 500) of claim 9, wherein the absorbent plug (220, 420) includes a plurality of fibers that can retain the anesthetic agent (222, 422) via absorption prior to contacting the target tissue.

11. The apparatus (200, 400, 500) of any one of claims 1 to 10, wherein, in a dimension transverse to a longitudinal axis of the apparatus (200, 400, 500), the hand piece (218) has a size that is larger than a size of the elongated shaft (212, 412) to provide a larger gripping surface.

12. The apparatus (200, 400, 500) of any one of claims 1 to 11, wherein the absorbent plug (220, 420) has a first cross-sectional diameter that is less than or equal to a cross-sectional diameter of the elongated shaft (212, 412) when the absorbent plug (220, 420) is in the compressed state, and wherein the absorbent plug (220, 420) has a second cross-sectional diameter that is greater than the cross-sectional diameter of the elongated shaft (212, 412) when the absorbent plug (220, 420) is in the uncompressed state.

13. The apparatus (200, 400, 500) of any one of claims 1 to 12, further comprising:
a removal member (226) coupled to the absorbent plug (220, 420) and extending from the absorbent plug (220, 420) to a location external to the nasal cavity (100).

14. The apparatus (200, 400, 500) of claim 13, wherein the removal member (226) comprises a soft material with sufficient tensile strength to provide for pulling the absorbent plug (220, 420) out of the nasal cavity (100).

15. The apparatus (200, 400, 500) of claim 13 or 14, wherein the removal member (226) is housed in a lumen or a cavity within the elongated shaft (212, 412) and/or the hand piece (218) prior to decoupling the elongated shaft (212, 412) from the absorbent plug (220, 420).

## Patentansprüche

1. Vorrichtung (200, 400, 500) zur Abgabe eines Anästhesiemittels (222, 422) an ein Zielgewebe in einer Nasenhöhle (100) eines Patienten, wobei die Vorrichtung (200, 400, 500) umfasst:
einen langgestreckten Schaft (212, 412) mit einem proximalen Ende (214, 414) und einem distalen Ende (216, 416);
einen an das distale Ende (216, 416) des länglichen Schafts (212, 412) gekoppelten Absorptionsstopfen (220, 420), wobei der Absorptionsstopfen (220, 420) dazu ausgebildet ist, in einem komprimierten Zustand ein erstes Volumen und in einem nicht komprimierten Zustand ein zweites Volumen einzunehmen, wobei das zweite Volumen größer ist als das erste Volumen und wobei der Absorptionsstopfen (220, 420) dazu ausgebildet ist, ein Anästhesiemittel (222, 422) zu speichern und das Anästhesiemittel (222, 422) an das Zielgewebe in der Nasenhöhle (100) durch Kontaktieren des Zielgewebes in dem nicht komprimierten Zustand abzugeben; und
eine Hülle (224), die den Absorptionsstopfen (220, 420) zumindest zum Teil umhüllt, wobei die Hülle (224) den Absorptionsstopfen (220, 420) in dem komprimierten Zustand hält, wenn sie zumindest zum Teil über dem Absorptionsstopfen (220, 420) positioniert ist, und wobei ein Entfernen der Hülle (224) es dem Absorptionsstopfen (220, 420) ermöglicht, sich in den nicht komprimierten Zustand auszudehnen;
**dadurch gekennzeichnet, dass** die Vorrichtung (200, 400, 500) ferner umfasst
ein an das proximale Ende (214, 414) des länglichen Schafts (212, 412) gekoppeltes Handstück (218), das dazu ausgebildet ist, ein Greifen und Manipulieren der Vorrichtung (200, 400, 500) zu erleichtern; und wobei das Handstück (218) und der längliche Schaft (212, 412) dazu ausgebildet sind, nach dem Entfernen der Hülle (224) von dem Absorptionsstopfen (220, 420) entkoppelt zu werden.

2. Vorrichtung (200) nach Anspruch 1, wobei der Absorptionsstopfen (220) bereits vor einem Einführen in die Nasenhöhle (100) zur Abgabe des Anästhesiemittels (222) mit dem Anästhesiemittel (222) getränkt ist.

3. Vorrichtung (200, 400, 500) nach Anspruch 1 oder 2, wobei die Hülle (224) relativ zu dem länglichen Schaft (212, 412) und dem Absorptionsstopfen (220, 420) versetzbar ist, wobei die Hülle (224) in einer Richtung von dem distalen Ende (216, 416) in Richtung des proximalen Endes (214, 414) aus einer ersten Position in eine zweite Position versetzbar ist,
wobei die Hülle (224) in der ersten Position zumindest einen Teil des Absorptionsstopfens (220, 420) derart umhüllt, dass sich der Absorptionsstopfen (220, 420) in dem komprimierten Zustand befindet, und
wobei die Hülle (224) den Absorptionsstopfen (220, 420) in der zweiten Position nicht umhüllt und sich der Absorptionsstopfen (220, 420) im nicht komprimierten Zustand befindet.

4. Vorrichtung (200, 400, 500) nach einem der Ansprüche 1 bis 3, wobei der Schaft (224) eine auflösbare Beschichtung umfasst, die den Absorptionsstopfen (220, 420) in dem komprimierten Zustand zumindest zum Teil umhüllt.

5. Vorrichtung (400, 500) nach einem der Ansprüche 1 bis 4, ferner umfassend:
ein sich durch den länglichen Schaft (412) und in den Absorptionsstopfen (420) hinein erstreckendes Lumen (428), wobei das Lumen (428) einen oder mehrere Öffnungen (430) innerhalb des Absorptionsstopfens (420) umfasst, der/die dazu ausgebildet ist/sind, das Anästhesiemittel (422) in den Absorptionsstopfen (420) abzugeben, um von dem Absorptionsstopfen (420) absorbiert zu werden.

6. Vorrichtung (400, 500) nach Anspruch 5, wobei der Absorptionsstopfen (420) dazu ausgebildet ist, sich in Erwiderung auf das Absorbieren des Anästhesiemittels (422) durch den Absorptionsstopfen (420) auszudehnen.

7. Vorrichtung (500) nach Anspruch 5 oder 6, ferner umfassend:
ein sich durch den länglichen Schaft (412) und in den Absorptionsstopfen hinein erstreckendes zweites Lumen (532), wobei das zweite Lumen (532) einen oder mehrere zweite Öffnungen (534) innerhalb des Absorptionsstopfens (420) umfasst, der/die dazu ausgebildet ist/sind, einen Sog anzulegen, um das Anästhesiemittel (422) aus dem Absorptionsstopfen (420) zu entfernen und den Absorptionsstopfen (420) aus einem nicht komprimierten Zustand in einen komprimierten Zustand zu schrumpfen.

8. Vorrichtung (200, 400, 500) nach einem der Ansprüche 1 bis 7, wobei der längliche Schaft (212, 412) ein verformbares und/oder flexibles Material umfasst.

9. Vorrichtung (200, 400, 500) nach einem der Ansprüche 1 bis 8, wobei der Absorptionsstopfen (220, 420) ein weiches und absorbierendes Material umfasst, das geeignet ist, das Anästhesiemittel (222, 422) vor dem Kontaktieren des Zielgewebes zu speichern.

10. Vorrichtung (200, 400, 500) nach Anspruch 9, wobei der Absorptionsstopfen (220, 420) eine Mehrzahl von Fasern aufweist, die das Anästhesiemittel (222, 422) durch Absorption vor dem Kontaktieren des Zielgewebes speichern können.

11. Vorrichtung (200, 400, 500) nach einem der Ansprüche 1 bis 10, wobei das Handstück (218) in einer Dimension quer zu einer Längsachse der Vorrichtung (200, 400, 500) eine Größe hat, die größer ist als eine Größe des länglichen Schafts (212, 412), um eine größere Greifoberfläche zu bieten.

12. Vorrichtung (200, 400, 500) nach einem der Ansprüche 1 bis 11, wobei der Absorptionsstopfen (220, 420) einen ersten Querschnittsdurchmesser hat, der kleiner oder gleich einem Querschnittsdurchmesser des länglichen Schafts (212, 412) ist, wenn sich der Absorptionsstopfen (220, 420) in dem komprimierten Zustand befindet, und wobei der Absorptionsstopfen (220, 420) einen zweiten Querschnittsdurchmesser hat, der größer ist als der Querschnittsdurchmesser des länglichen Schafts (212, 412), wenn sich der Absorptionsstopfen (220, 420) in dem nicht komprimierten Zustand befindet.

13. Vorrichtung (200, 400, 500) nach einem der Ansprüche 1 bis 12, ferner umfassend:
ein an den Absorptionsstopfen (220, 420) gekoppeltes und sich von dem Absorptionsstopfen (220, 420) an eine Stelle außerhalb der Nasenhöhle (100) erstreckendes Entfernungselement (226).

14. Vorrichtung (200, 400, 500) nach Anspruch 13, wobei das Entfernungselement (226) ein weiches Material mit ausreichender Reißfestigkeit umfasst, um ein Herausziehen des Absorptionsstopfens (220, 420) aus der Nasenhöhle (100) zu ermöglichen.

15. Vorrichtung (200, 400, 500) nach Anspruch 13 oder 14, wobei das Entfernungselement (226) vor dem Entkoppeln des länglichen Schafts (212, 412) von dem Absorptionsstopfen (220, 420) in einem Lumen oder einem Hohlraum innerhalb des länglichen Schafts (212, 412) und/oder des Handstücks (215) untergebracht ist.

## Revendications

1. Appareil (200, 400, 500) pour administrer un agent anesthésique (222, 422) à un tissu cible dans une cavité nasale (100) d'un patient, l'appareil (200, 400, 500) comprenant:
un arbre allongé (212, 412) ayant une extrémité proximale (214, 414) et une extrémité proximale (216, 416);
un bouchon absorbant (220, 420) accouplé à l'extrémité distale (216, 416) de l'arbre allongé (212, 412), dans lequel le bouchon absorbant (220, 420) est conçu pour occuper un premier volume dans un état comprimé et un second volume dans un état non comprimé et dans lequel le second volume est supérieur au premier volume, dans lequel le bouchon absorbant (220, 420) est conçu pour stocker un agent anesthésique (222, 422) et pour administrer l'agent anesthésique (222, 422) au tissu cible dans la cavité nasale (100) par mise en contact du tissu cible dans un état non comprimé; et
une gaine (224) recouvrant au moins partiellement le bouchon absorbant (220, 420), dans lequel la gaine (224) retient le bouchon absorbant (220, 420) dans un état comprimé lorsqu'elle est au moins partiellement positionnée sur le bouchon absorbant (220, 420), et dans lequel le retrait de la gaine (224) permet au bouchon absorbant (220, 420) de se dilater dans l'état non comprimé;
**caractérisé en ce que** l'appareil (200, 400, 500) comprenant en outre
une pièce à main (218) accouplée à l'extrémité proximale (214, 414) de l'arbre allongé (212, 414), dans lequel la pièce à main (218) est conçue pour faciliter la préhension et la manipulation de l'appareil (200, 400, 500); et dans lequel la pièce à main (218) et l'arbre allongé (212, 412) sont conçus pour être désaccouplés du bouchon absorbant (220, 420) après le retrait de la gaine (224).

2. Appareil (200) selon la revendication 1, dans lequel le bouchon absorbant (220) est pré-imprégné d'agent anesthésique (222) avant une insertion dans la cavité nasale (100) pour administrer l'agent anesthésique (222).

3. Appareil (200, 400, 500) selon l'une quelconque des revendications 1 ou 2, dans lequel la gaine (224) peut être translatée par rapport à l'arbre allongé (212, 412) et au bouchon absorbant (220, 420), dans lequel la gaine (224) peut être translatée dans une direction depuis l'extrémité distale (216, 416) en direction de l'extrémité proximale (214, 414) d'une première position à une seconde position,
dans lequel, dans la première position, la gaine (224) recouvre au moins une partie du bouchon absorbant (220, 420) de sorte que le bouchon absorbant (220, 420) soit dans l'état comprimé, et
dans lequel, dans la seconde position, la gaine (224) ne recouvre pas le bouchon absorbant (220, 420) et le bouchon absorbant (220, 420) est dans l'état non comprimé.

4. Appareil (200, 400, 500) selon l'une quelconque des revendications 1 à 3, la gaine (224) comprend un revêtement soluble recouvrant au moins partiellement le bouchon absorbant (220, 420) dans l'état comprimé.

5. Appareil (400, 500) selon l'une quelconque des revendications 1 à 4, comprenant en outre:
une lumière (428) s'étendant à travers l'arbre allongé (412) et dans le bouchon absorbant (420), dans lequel la lumière (428) comprend un ou plusieurs orifices (430) à l'intérieur du bouchon absorbant (420) conçus pour délivrer l'agent anesthésique (422) dans le bouchon absorbant (420) afin qu'il soit absorbé par le bouchon absorbant (420).

6. Appareil (200) selon la revendication 5, dans lequel le bouchon absorbant (420) est conçu pour se dilater de manière adaptée à l'agent anesthésique (422) absorbé par le bouchon absorbant (420).

7. Appareil (500) selon l'une quelconque des revendications 5 ou 6, comprenant en outre:
une seconde lumière (532) s'étendant à travers l'arbre allongé (412) et dans le bouchon absorbant (420), dans lequel la seconde lumière (532) comprend un ou plusieurs orifices (534) à l'intérieur du bouchon absorbant (420) conçus que l'agent anesthésique (422) soit retiré par aspiration du bouchon absorbant (420) et pour faire passer le bouchon absorbant (420) d'un état non comprimé à un état comprimé.

8. Appareil (200, 400, 200) selon l'une quelconque des revendications 1 à 7, dans lequel l'arbre allongé (212, 412) comprend un matériau malléable et/ou souple.

9. Appareil (200, 400, 500) selon l'une quelconque des revendications 1 à 8, dans lequel le bouchon absorbant (220, 420) comprend un matériau doux et absorbant apte à retenir l'agent anesthésique (222, 422) avant qu'il n'entre en contact avec le tissu cible.

10. Appareil (200, 400, 500) selon la revendication 9, dans lequel le bouchon absorbant (220, 420) comprend une pluralité de fibres qui peuvent retenir l'agent anesthésique (222, 422) par absorption avant qu'il n'entre en contact avec le tissu cible.

11. Appareil (200, 400, 500) selon l'une quelconque des revendications 1 à 10, dans lequel, dans une dimension transversale à un axe longitudinal de l'appareil (200, 400, 500), la pièce à main (218) a une taille qui est supérieure à une taille de l'arbre allongé (212, 412) pour fournir une surface de préhension plus grande.

12. Appareil (200, 400, 500) selon l'une quelconque des revendications 1 à 11, dans lequel le bouchon absorbant (220, 420) a un premier diamètre transversal qui est inférieur ou égal à un diamètre transversal de l'arbre allongé (212, 412) lorsque le bouchon absorbant (220, 420) est dans l'état comprimé, et dans lequel le bouchon absorbant (220, 420) a un second diamètre transversal qui est supérieur au diamètre transversal de l'arbre allongé (212, 412) lorsque le bouchon absorbant (220, 420) est dans l'état non comprimé.

13. Appareil (200, 400, 500) selon l'une quelconque des revendications 1 à 12, comprenant en outre:
un élément de retrait (226) accouplé au bouchon absorbant (220, 420) et s'étendant du bouchon absorbant (220, 420) à un emplacement à l'extérieur de la cavité nasale (100).

14. Appareil (200, 400, 500) selon la revendication 13, dans lequel l'élément de retrait (226) comprend un matériau doux ayant une résistance à la traction suffisante pour faire sortir le bouchon absorbant (220, 420) de la cavité nasale (100).

15. Appareil (200, 400, 500) selon la revendication 13 ou 14, dans lequel l'élément de retrait (226) est logé dans une lumière ou une cavité à l'intérieur de l'arbre allongé (212, 412) et/ou de la pièce à main (218) avant le désaccouplement de l'arbre allongé (212, 412) du bouchon absorbant (220, 420).
